(19) Europäisches Patentamt
European Patent Office
Office européen des brevets

(11) Publication number: 0 279 063 B1

(12) **EUROPEAN PATENT SPECIFICATION**

(45) Date of publication of patent specification: **09.10.91**

(51) Int. Cl.5: **C07D 333/20**, C07D 333/58, C07D 307/52, C07D 213/38, C07D 277/28, C07D 233/64, C07D 231/12, A61K 31/38

(21) Application number: **87118932.0**

(22) Date of filing: **21.12.87**

(54) **Allenyl amines.**

(30) Priority: **23.12.86 US 945460**

(43) Date of publication of application:
**24.08.88 Bulletin 88/34**

(45) Publication of the grant of the patent:
**09.10.91 Bulletin 91/41**

(84) Designated Contracting States:
**AT BE CH DE ES FR GB GR IT LI LU NL SE**

(56) References cited:
**EP-A- 0 186 915**
**EP-A- 0 187 390**

**CHEMICAL ABSTRACTS, vol. 104, 14 Apr. 1986, no. 129726m**

(73) Proprietor: **MERRELL DOW PHARMACEUTICALS INC.**
**2110 East Galbraith Road**
**Cincinnati Ohio 45215-6300(US)**

(72) Inventor: **McCarthy, James R.**
**100 Maxwell Lane**
**Zionsville Indiana 46077(US)**
Inventor: **Bargar, Thomas M.**
**125 Lynn Court**
**Zionsville Indiana 46077(US)**
Inventor: **Barney, Charlotte L.**
**4234 Eagle Bay West Drive**
**Indianapolis Indiana 46254(US)**
Inventor: **Matthews, Donald P.**
**1250 Meadowbrook Drive**
**Indianapolis Indiana 46240(US)**
Inventor: **Broersma, Robert J.**
**1233 Willow Way**
**Noblesville Indiana 46060(US)**

(74) Representative: **Sgarbi, Renato et al**
**GRUPPO LEPETIT S.p.A. Patent and Trademark Department 34, Via Roberto Lepetit**
**I-21040 Gerenzano (Varese)(IT)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 279 063 B1

## Description

This invention relates to a process for preparing allenyl amines, and the intermediates useful for this process.

More specifically, this invention relates to a process for preparing allenyl amines of the general formula

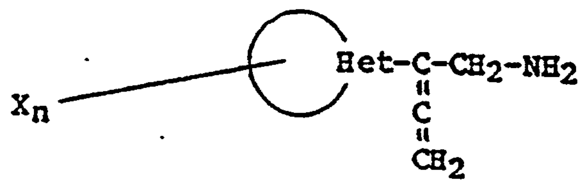

(I)

wherein Het is furyl, pyridyl, thiazolyl, imidazolyl, pyrazolyl, benzo/b/thienyl or a thienyl moiety; n is the integer 0, 1 or 2; each X independently is a substituent selected from ($C_1$-$C_6$)alkyl, halo, -O-($C_1$-$C_6$)alkyl,-S-($C_1$-$C_6$)alkyl, -SO-($C_1$-$C_6$)alkyl,-$SO_2$-($C_1$-$C_6$)alkyl,-$CO_2R$ and -$CH_2OR$, wherein halo is chloro, bromo or fluoro and R is H or ($C_1$-$C_6$)alkyl, and the therapeutically-acceptable acid addition salts thereof.

β-Methylene furanethanamines are described in EPA-186915 as possessing dopamine-β-hydroxylase (DBH)-inhibiting activity while β-methylenethiophenethaamines are described in EP-A-187390 for the same activity.

The Het moieties, which can optionally be substituted with 1-2 substituents as defined by X, are illustrated as follows:

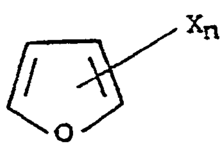

furyl

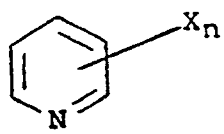

pyridyl

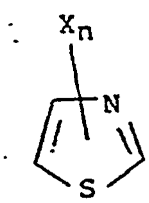

thiazolyl

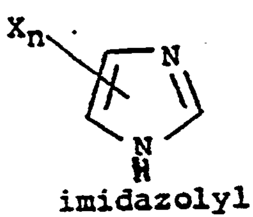

imidazolyl

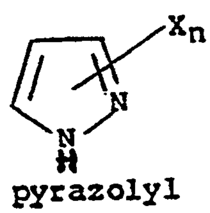

pyrazolyl

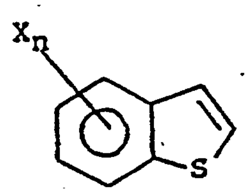

benzo/b/thienyl

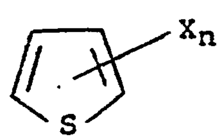

thienyl

wherein n and X are as above defined.

Where n is 2, the X substituents on each Het group can be the same or different, although they

preferably are the same. Preferably, n is O or 1. R is preferably H. The term "alkyl" in each occurrence means from to 1 to 6 carbon atoms, and includes methyl, ethyl, propyl, butyl, pentyl and hexyl groups, which can be straight- or branched-chain. Preferably, alkyl groups of from 1 to 4 carbon atoms are employed.

The following represent preferred embodiments of the compounds prepared by the process of invention:

(1) n is 0 or
(2) a is 1,
(3) n is 2;
(4) n is 1 and X is selected from the group consisting of halo and ($C_1$-$C_6$)alkyl; thienyl
(5) the embodiment of (4) wherein Het is thienyl or benzo[b]thienyl;
(6) the embodiment of (2) wherein X is -O- or -S-($C_1$-$C_6$)alkyl;
(7) the embodiment of (2) wherein X is -$CO_2$R;
(8) the embodiment of (2) wherein X is -$CH_2$OR;
(9) the embodiment of (1) wherein Het is thienyl;
(10) the embodiment of (2) wherein X is alkyl of from 1 to 4 carbon atoms, and
(11) the embodiment of (1) wherein Het is benzo[b]thienyl.

Illustrative examples of the compounds prepared by the process of this invention include $\beta$-ethenylidene-2-thiopheneethanamine,$\beta$-ethenylidene-2-furaneethanamine, $\beta$-ethenylidene-2-benzo-[b]-thiophene-ethanamine; $\beta$-ethenylidene-2-pyridineethanamine, $\beta$-ethenylidene-2-thiazoleethanamine, $\beta$-ethenylidene2-imidazoleethanamine,$\beta$-ethenylidene-3-pyrazoleethanamine $\beta$-ethenylidene-5-chloro-2-thiopheneethanamine,
$\beta$-ethenylidene-5-methoxy-2-thiopheneethanamine,
$\beta$-ethenylidene-5-carboxy-2-thiopheneethanamine,
$\beta$-ethenylidene-5-methylsulfinyl-2-thiopheneethanamine,
$\beta$-ethenylidene-5-methyl-2-furanethanamine,
$\beta$-ethenylidene-3-bromo-2-benzo[b]thiopheneethanamine,
$\beta$-ethenylidene-5-bromo-2-benzo[b]thiopheneethanamine,
$\beta$-ethenylidene-3,5-dibromo-2-benzo[b]thiopheneethanamine,
$\beta$-ethenylidene-5-chloro-2-pyridineethanamine,
$\beta$-ethenylidene-5-methyl-2-thiazoleethanamine,
$\beta$-ethenylidene-5-methoxy-2-imidazoleethanamine and
$\beta$-ethenylidene-5-chloro-2-pyrazoleethanamine
and the therapeutically acceptable acid addition salts thereof.

Representative salts are those salts formed with nontoxic organic or inorganic acids, such as, for example, those formed from the following acids: hydrochloric, hydrobromic, sulfonic, sulfuric, phosphoric, nitric, maleic, fumaric, benzoic, ascorbic, succinic, methanesulfonic, acetic, propionic, tartaric, citric, lactic, malic, mandelic, cinnamic, palmitic, itaconic and benzenesulfonic.

The process of the invention allows the ready preparation of the allenyl amines (I) by-a series of reaction steps which are illustrated by the following reaction scheme:

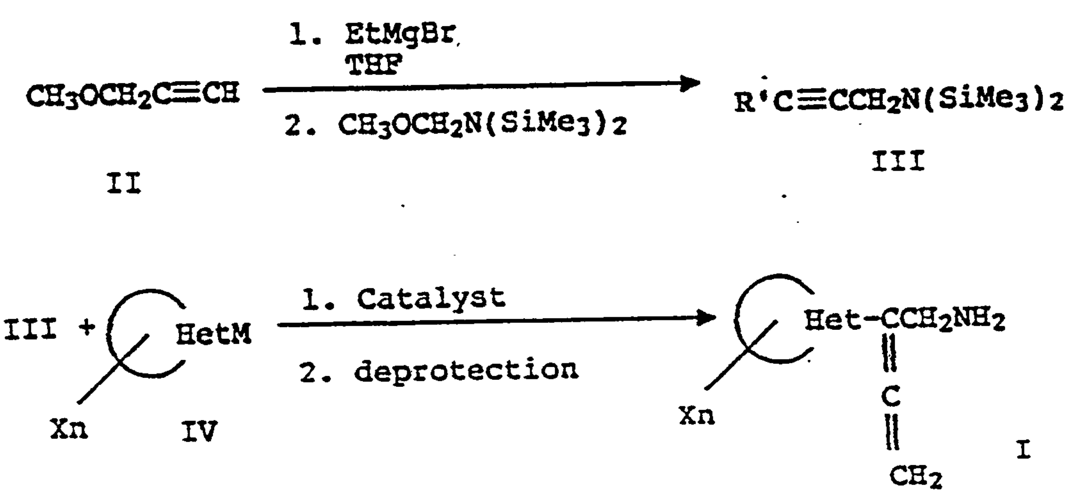

In essence, the foregoing reaction scheme shows that the allenyl amines (I) are prepared by the novel reaction of a protected amine, N,N-bis(trimethylsilyl)-4-alkoxy-2-butynylamine (III), with a metallo-organic compound of formula (IV) wherein Het, X and n are as defined above and M is a metal containing moiety, such as for example, -MgBr, a trimethylstannyl or a tri-n-butylstannyl group. In formula III, R′ is an alkoxymethyl group of 1-4 carbon atoms, and is preferably methoxymethyl. The reaction is carried out in an inert solvent, such as diethylether, at room temperature in the presence of a nickel catalyst, such as 1,3-bis-(diphenylphosphinopropane)-nickel(II)chloride [henceforth $NiCl_2$(dppp)], to yield the silyl-protected amines (III). The reaction product is then treated with a deprotecting agent, e.g., such as ammonium hydroxide and silica gel (column chromatography), or ethanolic hydrochloric acid or sodium fluoride, to remove the silyl protecting groups and give the desired product (I). The free bases can be converted into acid addition salts, or the acid addition salts can be converted into the free bases by conventional chemical methodology.

The trimethylsilyl starting material (III) is prepared by the addition of ethylmagnesium bromide to a mixture of methylpropargyl ether (II) and tetrahydrofuran (THF), after which N,N-bis(trimethylsilyl) methoxymethylamine is added. After dilution with ether, filtration through celite and washing with sodium hydroxide, Kugelrohr distillation yields bis(trimethylsilyl) protected 4-methoxy-2-butynylamine (III).

When the metallo-organic starting compound in the above reaction is a Grignard reagent, it is obtained by converting the appropriate brominated heterocycle (see Het above), for example, 2-bromothiophene, by reactions with magnesium according to standard Grignard conditions to yield, 2-thienylmagnesium bromide; or by converting the appropriate lithiated heterocycle, for example 2-benzo[b]thienyllithium lithium, with magnesium bromide to yield, 2-benzo[b]thienylmagnesium bromide according to standard Grignard conditions.

When M in the above reaction is a tin derivative, it can be obtained in a manner consistent with the procedure of T. Bailey, Tetrahedron Letters, 27, 37, 4407 (1986).

The foregoing reaction scheme is further illustrated by the following specific exemplifications.

## EXAMPLE 1

### N,N-bis(trimethylsilyl) 4-methoxy-2 butynylamine (III)

Methylpropargyl ether (36.4g,0.52M) and dry tetrahydrofuran (300 ml) were added to a dry 100 ml 3-necked flask with mechanical stirrer, thermometer, and addition funnel with a nitrogen bubbler. The solution was cooled in an ice bath and ethylmagnesium bromide (262 ml, 0.52M) was added dropwise with stirring. The reaction mixture was stirred at room temperature for 10 minutes and N,N-bis(trimethylsilyl) methoxymethylamine was added. The reaction mixture was heated at reflux for 16 hours until complete by C G analysis. The thick slurry was diluted with ether, filtered through a celite pad and the filtrate washed with 1 liter 30% NaOH. The product was dried over $K_2CO_3/Na_2SO_4$ and concentrated into an oil. Kugelrohr distillation at 80 °C (2 mmHg) gave 91 g. (77%) of III.

## EXAMPLE 2

### β-Ethenylidene-2-thiopheneethanamine

$NiCl_2$ (dppp) catalyst (170 mg; 3M %) was added to dry ether (30 ml) in a 100 ml 3-necked flask with mechanical stirrer, thermometer and nitrogen bubbler. III (2.47g, 10.2 mM) was added via syringe with stirring and immediately thereafter 3M 2-thienylmagnesium bromide (7 ml, 21 mM) was added via syringe. After stirring at room temperature for 20 hours the reaction was complete as shown by C G Analysis. The silylated product was isolated by shaking with dilute $NH_4OH$ and extraction into ethylacetate (three times, each into 100 ml). After drying over $K_2CO_3$ and concentration, 2.68 g crude product was obtained. Purification and desilylation by flash chromatography (300 g silica gel, eluted with $CHCl_3$, then $CHCl_3$:MeOH:conc. $NH_4OH$ 100:10:1) gave 1.22 g (87%) of the desired product as a light tan oil after drying under high vacuum.

Treatment of an etheral solution of the free base with one equivalent of 1M ethanolic p-toluenesulfonic acid or with one equivalent of an ethanolic solution of oxalic acid gave the tosylate and oxalate salts respectively. The tosylate salt was a 76% conversion from the free base, Mp 126-127 °C (isopropanol). The oxalate salt was a 72% conversion from the free base, Mp 188-190 °C (isopropanol).
Anal. Calc'd for $C_8H_9NS$: C, 55.71; H, 5.30; N, 5.30.
Found: C, 55.75; H,5.44; N, 4.28.

EXAMPLE 3

β-Ethenylidene-2-benzo[b]thiophene-ethanamine

2-Benzo[b]thienylmagnesium bromide was prepared by adding butyllithium (5.1 ml, 15.8 mM) to benzo-[b]thiophene (1.7 g., 12.7 mM) in a 3-necked, 100 ml flask with septum stir bar, thermometer, and nitrogen inlet, in the presence of ether (15 ml), allowing reaction to reflux about 15 minutes, and then cooling to 0° C in ice. Magnesium bromide etherate (12.7 mM) was added all at once and was allowed to warm to 20° C over about 15 minutes. Then $NiCl_2$(dppp) (30 mg) was added and, following the procedure described in Example 2, III (12.7 mM) was added and stirred overnight to produce the crude silylated product, the reaction being complete by G C analysis after that time. 1M ethanolic hydrochloride (0.9 equivalent), was added to the etheral solution to yield 1.7 g (72%) of the desired product as a pale yellow solid, Mp 235° C (dec.) (EtOH).

Anal. Calc'd for $C_{12}H_{11}NS.HCl$: C, 60.63; H, 5.09; N, 5.89. Found: C, 60.86; H, 5.21; N, 5.65.

The allenylamines obtained by the process of this invention (I) are dopamine-β-hydroxylase (DBH) inhibitors of a mechanism-based nature, inactivation being time dependent. The enzyme is inactivated directly, i.e., at the active site and thus the compounds of formula I are useful as therapeutic agents in the treatment of hypertension. An embodiment if this invention thus comprises a process for producing a medicament for antihypertensive use which comprises preparing a compound of formula (I) by the process of this invention and then mixing said compound with a pharmaceutically acceptable carrier.

The DBH inhibitory properties of the compounds obtained by the process of this invention can readily be determined by standard and well known procedures such as those procedures set forth in U.S. Patent 4,415,591. Determination of whether the DBH inhibition demonstrates time-dependent kinetics is exemplified by a procedure wherein enzymatic oxygenation by DBH is determined in aqueous solution in the presence of molecular oxygen, an electron donor such as ascorbate, and the necessary cofactors for the enzyme at a pH of 4.5 to 5.5, preferably pH 5.0, and at a temperature of 20° C to 40° C, preferably 37° C. The test compound is added at the desired concentration, and the system is incubated. At different time intervals aliquots are taken and DBH activity is measured using tyramine as the substrate. The reaction is followed by measuring oxygen uptake using a polarographic electrode and an oxygen monitor by the method of S. May et al., J. Biol. Chem. 256, 2258 (1981). In tests utilizing the above described procedure, the DBH inhibitory activity of the test compound increased as a function of the time of incubation as indicated in Table I:

TABLE I

DBH INHIBITORY ACTIVITY - *IN VITRO*

| Compound | Concentration | t 1/2* |
|---|---|---|
| β-ethenylidene - 2-thiopheneethanamine | 1mM | 78.2 min +/-1.1 |

*t 1/2 = time required for 50% inhibition

The ability of compounds obtained by the process of this invention to lower blood pressure can be determined *in vivo* using hypertensive rats according to standard and well known procedures. The test compound is administered intraperitoneally (ip) to conscious rats and the blood pressure monitored continuously. Since DBH is a major enzyme in the synthetic pathway of the catecholamines, it would be expected that the presence of an inhibitor would act to decrease the amount of catecholamines produced, and thereby have an antihypertensive effect. When tested for antihypertensive activity in the above manner, a decrease in mean blood pressure for a period of time was as indicated for the test compound, as set forth

in Table II:

TABLE II

ANTIHYPERTENSIVE ACTIVITY - *IN VIVO*

| Compound | Dose | Maximum % Change in mean Blood Pressure | Duration |
|---|---|---|---|
| β-ethenylidene-2-thiopheneethanamine | 50 mg/kg (ip) | 15.6 | 10 hr. |

Thus, based upon this and other standard laboratory techniques known to evaluate DBH inhibitors, by standard toxicity tests and by standard pharmacological assays for the determination of antihypertensive activity in mammals, and by comparison of these results with known antihypertensive agents the effective antihypertensive the process dosage of the compounds obtained by the process of this invention can readily be determined. In general, the effective antihypertensive results can be achieved at a dose of about 5 to about 100 mg per kilogram of body weight per day. Of course, the specific initial and continuing dosage regimen for each patient will vary according to the nature and severity of the hypertension as determined by the attending diagnostician.

In their function as therapeutically useful compounds, it is advantageous to administer the compounds to the host animal as a composition in admixture with a pharmaceutically acceptable carrier suitable for internal administration, said carrier constituting a major portion of the admixture. Such preparations will be apparent to those skilled in the art and may be in such forms as, for example, tablets, capsules and suppositories, or in liquid forms, as for example, elixirs, emulsions, sprays and injectables. In the formulation of pharmaceutical preparations there can be employed such substances which do not react with active substances as, for example, water, gelatin, lactose, starches, magnesium sterate, talc, vegetable oils, benzyl alcohols, gums, polyalkylene glycols, petroleum jelly and the like. The active ingredient of such pharmaceutical preparations is preferably present in the preparation in such proportions by weight that the proportion by weight of the active ingredient to be administered lies between 0.1% and 50%.

## Claims

1. A process for preparing a compound of the general formula

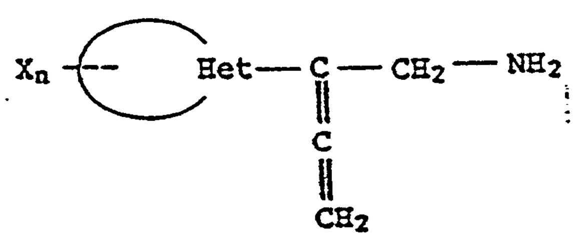

(I)

wherein Het is a furyl, pyridyl, thiazolyl, imidazolyl, pyrazolyl,benzo[b]thienyl or a thienyl moiety; n is the integer 0, 1 or 2; each X independently is a substituent selected from $(C_1\text{-}C_6)$alkyl, chloro-, bromo-, fluoro-, $-O-(C_1\text{-}C_6)$alkyl, $-S-(C_1\text{-}C_6)$alkyl, $-SO-(C_1\text{-}C_6)$alkyl, $-SO_2-(C_1\text{-}C_6)$alkyl, $-CO_2R$ and $-CH_2OR$

wherein R is hydrogen or ($C_1$-$C_6$)alkyl, and the therapeutically acceptable acid addition salts thereof, which comprises reacting a protected amine compound of the general formula

$R'C{\equiv}CCH_2N(SiMe_3)_2$ (III)

wherein R' is a ($C_1$-$C_4$)alkoxymethyl group, with a metallo-organic compound of the general formula

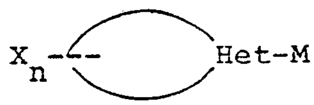 (IV)

wherein Het, X, and n are as above defined, and M is a metal containing moiety, in the presence of an inert solvent and a nickel catalyst, and further treating the resulting product bearing a silyl protected amine group with a deprotecting agent.

**2.** A process according to Claim 1 for preparing a compound of the genaral formula

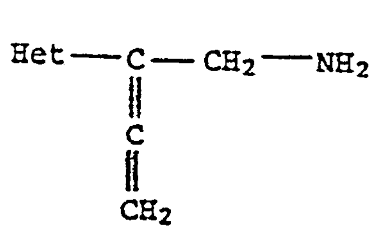

wherein Het is as defined in Claim 1, which comprises reacting a protected amine compound of the general formula

$R'C{\equiv}CH_2N(SiMe_3)_2$

with a metallo-organic compound of the general formula

Het-M

wherein Het is as above defined, R' is a ($C_1$-$C_4$)alkoxymethyl group and M is a metal containing moiety, in the presence of an inert solvent and a nickel catalyst, and further treating the resulting product bearing a silyl protected amine group with a deprotecting agent.

**3.** A process according to Claim 1 for preparing a compound of the general formula

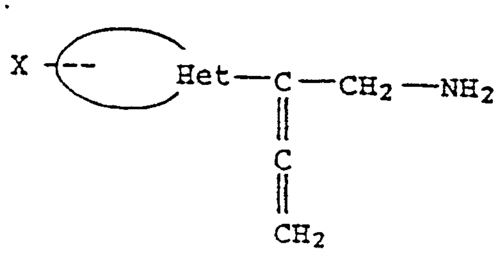

wherein X and Het are as defined in Claim 1, which comprises reacting a protected amine compound of the general formula

$R'C{\equiv}CCH_2N(SiMe_3)_2$

with a metallo-organic compound of the general formula

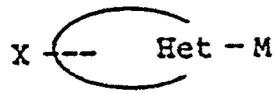

wherein X and Het are, as above defined, R' is a $(C_1-C_4)$alkoxymethyl group and M is a metal containing moiety, in the presence of an inert solvent and a nickel catalyst, and further treating the resulting product bearing a silyl protected amine group with a deprotecting agent.

**4.** A process according to Claim 1 for preparing a compound of the general formula

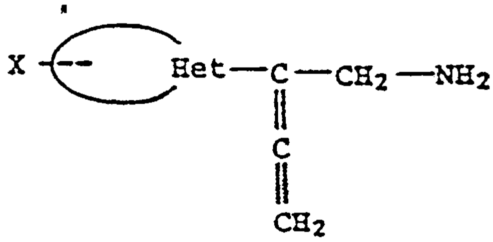

wherein X is selected from the group consisting of chloro-, bromo-, fluoro- and $(C_1-C_6)$alkyl, and Het is as defined in Claim 1, which comprises reacting a protected amine compound of general formula

$R'C{\equiv}CCH_2N(SiMe_3)_2$

with a metallo-organic compound of the genaral formula

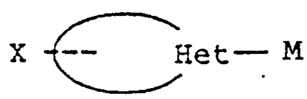

wherein X and Het are as above defined, R'is a $(C_1-C_4)$alkoxymethyl group and M is a metal containing moiety in the presence of an inert solvent and a nickel catalyst, and further treating the resulting product bearing a silyl protected amine group with a deprotecting agent.

**5.** A process according to Claim 1 for preparing a compound of the general formula

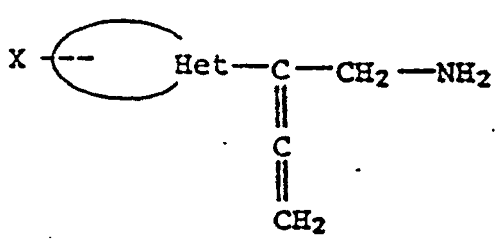

wherein X is -O-$(C_1-C_6)$alkyl, or -S-$(C_1-C_6)$alkyl,
and Het is as defined in Claim 1, which comprises reacting a protected amine compound of the general formula

$R'C{\equiv}CCH_2N(SiMe_3)_2$

with a metallo-organic compound of the general formula

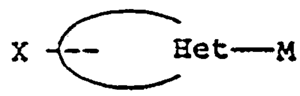

wherein X and Het are as above defined R' is a $(C_1\text{-}C_4)$alkoxymethyl group and M is a metal containing moiety, in the presence of an inert solvent and a nickel catalyst, and further treating the resulting product bearing a silyl protected amine group with a deprotecting agent.

6. A process according to Claim 1 for preparing a compound of the general formula

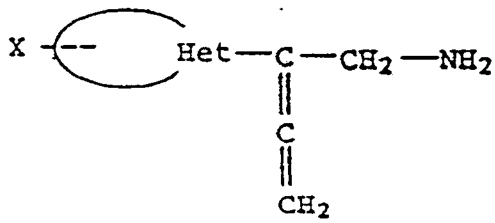

wherein X is $-CO_2R$ or $-CH_2OR$ and R and Het are as defined in Claim 1, which comprises reacting a protected amine compound of the general formula

$R'C{\equiv}CCH_2N(SiMe_3)_2$

with a metallo-organic compound of the general formula

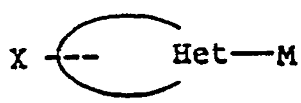

wherein X and Het are as above defined, R' is a $(C_1\text{-}C_4)$alkoxymethyl group and M is a metal containing moiety, in the presence of an inert solvent and a nickel catalyst, and further treating the resulting product bearing a silyl protected amine group with a deprotecting agent.

7. A prccess according to Claim 1 for preparing ß-ethenylidene-2-thiopheneethanamine which comprises reacting N,N-bis(trimethylsilyl) 4-methoxy-2-butynylamine with 2-thienylmagnesium bromide in the presence of an inert solvent and a nickel catalyst, and further treating the resulting product bearing a silyl protected amine group with a deprotecting agent.

8. A process according to Claim 1 for preparing β-ethenylidene-2-benzo[b]thiopheneethanamine which comprises reacting N,N-bis(trimethylsilyl) 4-methoxy-2-butynylamine with 2-benzo[b]thienylmagnesium bromide in the presence of an inert solvent and a nickel catalyst, and further treating the resulting product bearing a silyl protected amine group with a deprotecting agent.

9. A process for producing a medicament for antihypertensive use which comprises preparing a compound of the formula(I) by the process according to any of claims 1 to 8 and then mixing said compound with a pharmaceutically acceptable carrier.

## Revendications

1. Procédé pour préparer un composé de formule générale

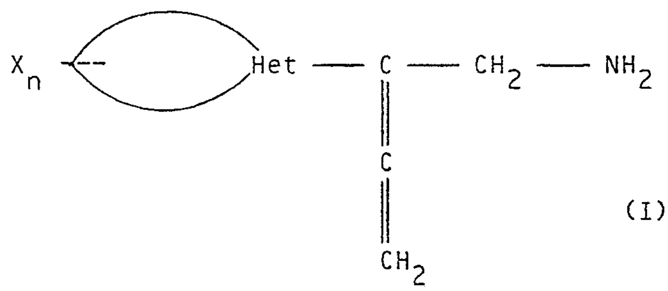

(I)

dans laquelle Het est un fragment furyle, pyridyle, thiazolyle, imidazolyle, pyrazolyle, benzo[b]thiényle ou thiényle ; n est un entier valant 0, 1 ou 2 ; chaque X indépendamment est un substituant choisi parmi alkyle en $C_1$-$C_6$ chloro, bromo, fluoro, -O-alkyle en $C_1$-$C_6$, -S-alkyle en $C_1$-$C_6$, -SO-alkyle en $C_1$-$C_6$, -$SO_2$-alkyle en $C_1$-$C_6$, -$CO_2$R et -$CH_2$OR où R est un hydrogène ou un alkyle en $C_1$-$C_6$, et leurs sels d'addition d'acides convenant en thérapeutique, qui comprend la réaction d'une amine protégée de formule générale

$R'C\equiv CCH_2N(SiMe_3)_2$ (III)

dans laquelle R' est un groupe (alcoxy en $C_1$-$C_4$)méthyle, avec un composé organométallique de formule qénérale

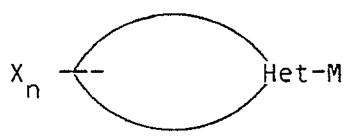

(IV)

dans laquelle Het, X et n sont comme définis ci-dessus et M est une fraction contenant un métal, en présence d'un solvant inerte et d'un catalyseur au nickel, et de plus le traitement du produit obtenu, portant un groupe amine silyl-protégé, avec un agent de déprotection.

**2.** Procédé selon la revendication 1 pour la préparation d'un composé de formule générale

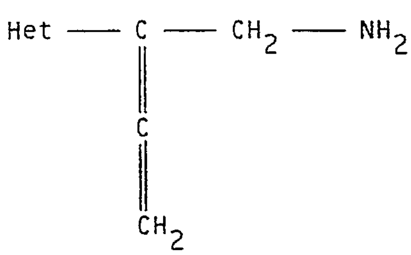

dans laquelle Het est comme défini dans la revendication 1, qui comprend la réaction d'une amine protégée de formule générale

$R'C\equiv CCH_2N(SiMe_3)_2$

avec un composé organométallique de formule générale

Het-M

dans lesquelles Het est comme défini ci-dessus, R' est un groupe (alcoxy en $C_1$-$C_4$) méthyle et M est une fraction contenant un métal, en présence d'un solvant inerte et d'un catalyseur au nickel, et de plus le traitement du produit obtenu, portant un groupe amine silyl-protégé, avec un agent de déprotection.

3. Procédé selon la revendication 1, pour préparer un composé de formule générale

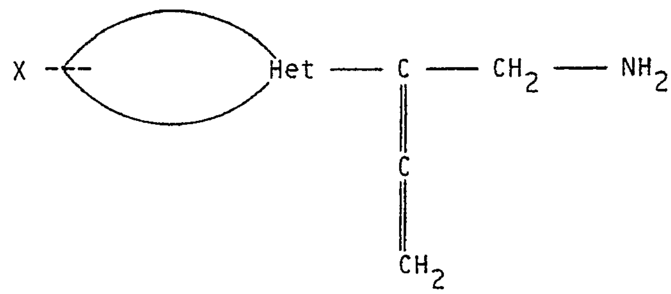

dans laquelle X et Het sont comme définis dans la revendication 1, qui comprend la réaction d'une amine protégée de formule générale

$R'C{\equiv}CCH_2N(SiMe_3)_2$

avec un composé organométallique de formule générale

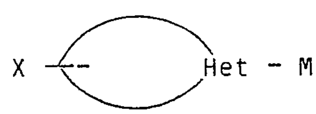

dans lesquelles X et Het sont comme définis ci-dessus, R' est un groupe (alcoxy en $C_1$-$C_4$)méthyle et M est une fraction contenant un métal, en présence d'un solvant inerte et d'un catalyseur au nickel, et de plus le traitement du produit obtenu, portant un groupe amine silyl-protégé, avec un agent de déprotection.

4. Procédé selon la revendication 1 pour la préparation d'un composé de formule générale

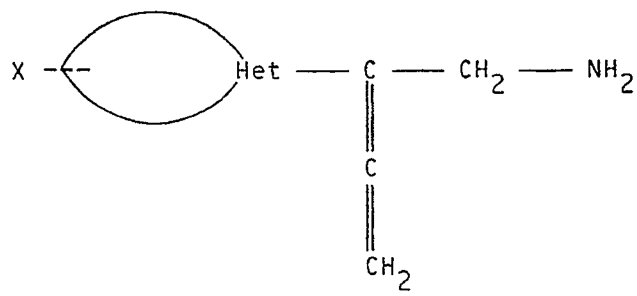

dans laquelle X est choisi parmi chloro, bromo, fluoro et alkyle en $C_1$-$C_6$, et Het est comme défini dans la revendication 1, qui comprend la réaction d'une amine protégée de formule générale

$R'C{\equiv}CCH_2N(SiMe_3)_2$

avec un composé organométallique de formule générale

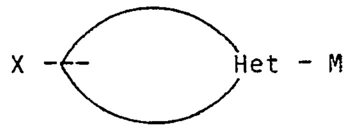

dans lesquelles X et Het sont comme définis ci-dessus, R' est un groupe (alcoxy en $C_1$-$C_4$)méthyle et

M est une fraction contenant un métal, en présence d'un solvant inerte et d'un catalyseur au nickel, et de plus le traitement du produit obtenu, portant un groupe amine silyl-protégé, avec un agent de déprotection.

5. Procédé selon la revendication 1 pour la préparation d'un composé de formule générale

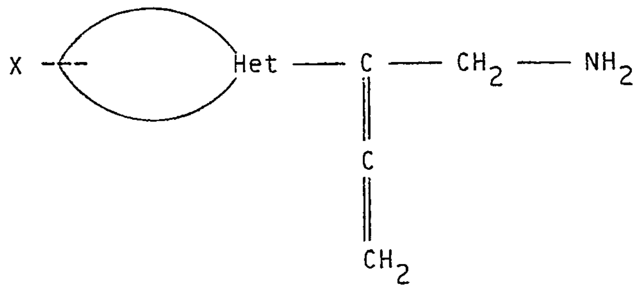

dans laquelle X est un -O-alkyle en $C_1$-$C_6$ ou un S-alkyle en $C_1$-$C_6$ et Het est comme défini dans la revendication 1, qui comprend la réaction d'une amine protégée de formule générale

$R'C{\equiv}CCH_2N(SiMe_3)_2$

avec un composé organométallique de formule générale

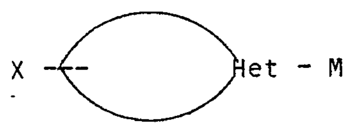

dans lesquelles X et Het sont comme définis ci-dessus, R' est un groupe (alcoxy en $C_1$-$C_4$)méthyle et M est une fraction contenant un métal, en présence d'un solvant inerte et d'un catalyseur au nickel, et de plus le traitement du produit obtenu, portant un groupe amine silyl-protégé, avec un agent de déprotection.

6. Procédé selon la revendication 1 pour préparer un composé de formule générale

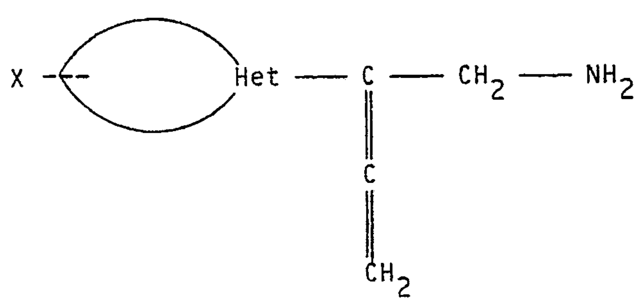

dans laquelle X est $-CO_2R$ ou $-CH_2OR$, et R et Het sont comme définis dans la revendication 1, qui comprend la réaction d'une amine protégée de formule générale

$R'C{\equiv}CCH_2N(SiMe_3)_2$

avec un composé organométallique de formule générale

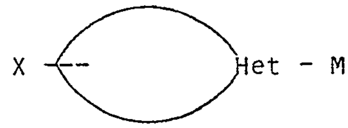

dans lesquelles X et Het sont comme définis ci-dessus, R' est un groupe (alcoxy en $C_1$-$C_4$)méthyle et M est une fraction contenant un métal, en présence d'un solvant inerte et d'un catalyseur au nickel, et de plus le traitement du produit obtenu, portant un groupe amine silyl-protégé, avec un agent de déprotection.

7. Procédé selon la revendication 1 pour préparer la $\beta$-éthénylidène-2-thiophène-éthanamine, qui comprend la réaction de la N,N-bis(triméthylsilyl)-4-méthoxy-2-butynylamine avec le bromure de 2-thiénylmagnésium en présence d'un solvant inerte et d'un catalyseur au nickel, et de plus le traitement du produit obtenu portant un groupe amine silyl-protégé, avec un agent de déprotection.

8. Procédé selon la revendication 1 pour préparer la $\beta$-éthénylidène-2-benzo[b]thiophène-éthanamine, qui comprend la réaction de la N,N-bis(triméthylsilyl)-4-méthoxy-2-butynylamine avec le bromure de 2-benzo[b]thiénylmagnésium en présence d'un solvant inerte et d'un catalyseur au nickel, et de plus le traitement du produit obtenu, portant un groupe amine silyl-protégé, avec un agent de déprotection.

9. Procédé pour produire un médicament à usage antihypertensif, qui comprend la préparation d'un composé de formule (I) selon le procédé de l'une quelconque des revendications 1 à 8, puis le mélange dudit composé avec un véhicule pharmaceutiquement acceptable.

## Patentansprüche

1. Verfahren zur Herstellung einer Verbindung der allgemeinen Formel

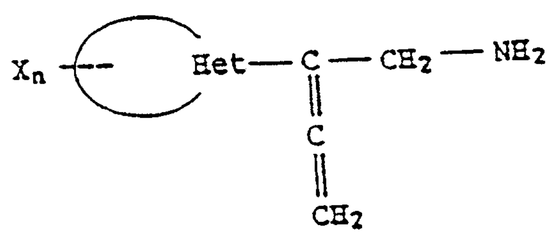

(I)

in der Het eine Furyl-, Pyridyl-, Thiazolyl-, Imidazolyl-, Pyrazolyl-, Benzo[b]thienyl- oder eine Thienylgruppe bedeutet, n die ganze Zahl 0, I oder 2 bedeutet, X jeweils unabhängig einen Substituenten aus ($C_1$-$C_6$)-Alkylresten, Chlor-, Brom-, Fluoratomen, -O-($C_1$-$C_6$)-Alkyl-, -S-($C_1$-$C_6$)-Alkyl-, -SO-($C_1$-$C_6$)-Alkyl-, -$SO_2$-($C_1$-$C_6$)-Alkylresten- oder den Resten -$CO_2R$ und -$CH_2OR$ bedeutet, in denen R ein Wasserstoffatom oder einen ($C_1$-$C_6$)-Alkylrest darstellt und die therapeutisch verträglichen Säureadditionssalze davon, umfassend - Umsetzung einer geschützten Aminoverbindung der allgemeinen Formel

$R'C{\equiv}CCH_2N(SiMe_3)_2$ (III)

in der R' einen ($C_1$-$C_4$)-Alkoxymethylrest bedeutet, mit einer metallorganischen Verbindung der allgemeinen Formel IV

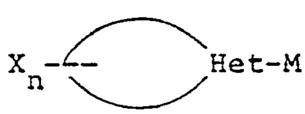

(IV)

in der Het, X und n wie vorstehend definiert sind und M einen metallhaltigen Rest darstellt, in Gegenwart eines inerten Lösungsmittels und eines Nickelkatalysators und anschließender Umsetzung des erhaltenen Produkts, das mit einer Silylrest-geschützten Aminogruppe versehen ist, mit einem Schutzgruppen entfernenden Mittel.

**2.** Verfahren nach Anspruch 1 zur Herstellung einer Verbindung der allgemeinen Formel

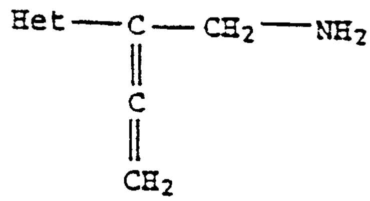

in der Het wie in Anspruch 1 definiert ist, umfassend - die Umsetzung einer geschützten Aminoverbindung der allgemeinen Formel

$R'C\equiv CCH_2N(SiMe_3)_2$

mit einer metallorganischen Verbindung der allgemeinen Formel

Het-M

in der Het wie vorstehend definiert ist, R' einen ($C_1$-$C_4$)-Alkoxymethylrest bedeutet und M einen metallhaltigen Rest darstellt, in Gegenwart eines inerten Lösungsmittels und eines Nickelkatalysators und anschließender Umsetzung des erhaltenen Produkts, das mit einer Silylrest-geschützten Aminogruppe versehen ist, mit einem Schutzgruppen entfernenden Mittel.

**3.** Verfahren nach Anspruch 1 zur Herstellung einer Verbindung der allgemeinen Formel

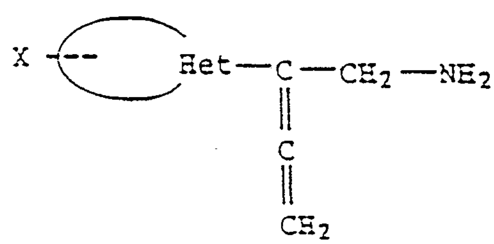

in der X und Het wie in Anspruch 1 definiert sind, umfassend - die Umsetzung einer geschützten Aminoverbindung der allgemeinen Formel

$R'C\equiv CCH_2N(SiMe_3)_2$

mit einer metallorganischen Verbindung der allgemeinen Formel

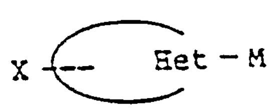

wobei X und Het wie vorstehend definiert sind, R' einen ($C_1$-$C_4$)-Alkoxymethylrest bedeutet und M einen metallhaltigen Rest darstellt, in Gegenwart eines inerten Lösungsmittels und eines Nickelkatalysators und anschließender Umsetzung des erhaltenen Produkts, das mit einer Silylrest-geschützten Aminogruppe versehen ist, mit einem Schutzgruppen entfernenden Mittel.

4. Verfahren nach Anspruch 1 zur Herstellung einer Verbindung der allgemeinen Formel

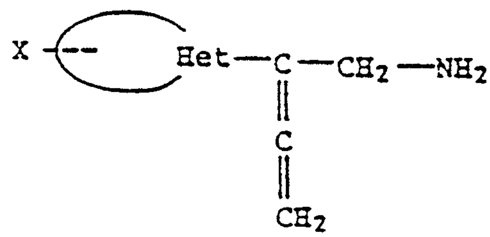

in der X aus der Gruppe Chlor-, Brom-, Fluoratome und ($C_1$-$C_6$)-Alkylreste ausgewählt ist und Het wie in Anspruch 1 definiert ist, umfassend - Umsetzung einer geschützten Aminoverbindung der allgemeinen Formel

$R'C{\equiv}CCH_2N(SiMe_3)_2$

mit einer metallorganischen Verbindung der allgemeinen Formel

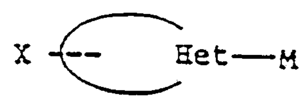

wobei X und Het wie vorstehend definiert sind, R' einen ($C_1$-$C_4$)-Alkoxymethylrest bedeutet und M einen metallhaltigen Rest darstellt, in Gegenwart eines inerten Lösungsmittels und eines Nickelkatalysators und anschließender Umsetzung des erhaltenen Produkts, das mit einer Silylrest-geschützten Aminogruppe versehen ist, mit einem Schutzgruppen entfernenden Mittel.

5. Verfahren nach Anspruch 1 zur Herstellung einer Verbindung der allgemeinen Formel

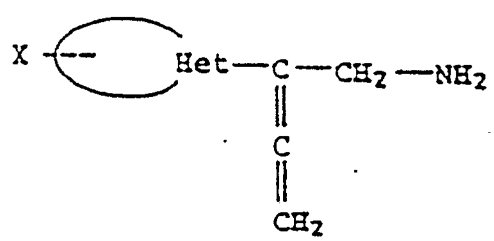

in der X einen -O-($C_1$-$C_6$)-Alkylrest oder -S-($C_1$-$C_6$)-Alkylrest bedeutet und Het wie in Anspruch 1 definiert ist, umfassend - Umsetzung einer geschützten Aminoverbindung der allgemeinen Formel

$R'C{\equiv}CCH_2N(SiMe)_2$

mit einer metallorganischen Verbindung der allgemeinen Formel

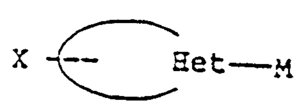

in der X und Het wie vorstehend definiert sind, R' einen ($C_1$-$C_4$)-Alkoxymethylrest und M einen metallhaltigen Rest bedeutet, in Gegenwart eines inerten Lösungsmittels und eines Nickelkatalysators

und anschließender Umsetzung des erhaltenen Produkts, das mit einer Silylrest-geschützten Aminogruppe versehen ist, mit einem Schutzgruppen entfernenden Mittel.

6. Verfahren nach Anspruch 1 zur Herstellung einer Verbindung der allgemeinen Formel

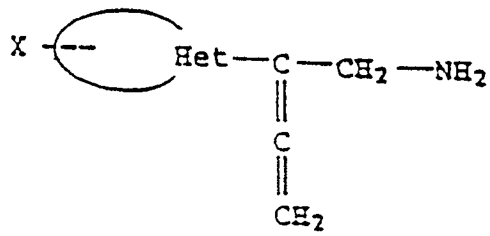

in der X die Gruppe $-CO_2R$ oder $-CH_2OR$ bedeutet und R und Het wie in Anspruch 1 definiert sind, umfassend - die Umsetzung einer geschützten Aminoverbindung der allgemeinen Formel

$R'C{\equiv}CCH_2N(SiMe_3)_2$

mit einer metallorganischen Verbindung der allgemeinen Formel

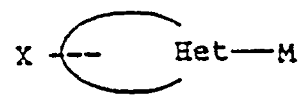

in der X und Het wie vorstehend definiert sind, R' einen $(C_1\text{-}C_4)$-Alkoxymethylrest bedeutet und M einen metallhaltigen Rest darstellt, in Gegenwart eines inerten Lösungsmittels und eines Nickelkatalysators und anschließender Umsetzung des erhaltenen Produkts, welches mit einer Silylrest-geschützten Aminogruppe versehen ist, mit einem Schutzgruppen entfernenden Mittel.

7. Verfahren nach Anspruch 1 zur Herstellung von β-Ethenyliden-2-thiophenethanamin umfassend - die Umsetzung von N,N-Bis(trimethylsilyl)-4-methoxy-2-butinylamin mit 2-Thienylmagnesiumbromid in Gegenwart eines inerten Lösungsmittels und eines Nickelkatalysators und anschließender Umsetzung des erhaltenen Produkts, das mit einer Silylrest-geschützten Aminogruppe versehen ist, mit einem Schutzgruppen entfernenden Mittel.

8. Verfahren nach Anspruch 1 zur Herstellung von β-Ethenyliden-2-benzo[b]thiophenethanamin umfassend - Umsetzung von N,N-Bis(trimethylsilyl)-4-methoxy-2-butinylamin mit 2-Benzo[b]-thienylmagnesiumbromid in Gegenwart eines inerten Lösungsmittels und eines Nickelkatalysators und anschließender Umsetzung des erhaltenen Produkts, das mit einer Silylrest-geschützten Aminogruppe versehen ist, mit einem Schutzgruppen entfernenden Mittel.

9. Verfahren zur Herstellung eines Arzneimittels zur Behandlung von Bluthochdruck, umfassend - Herstellung einer Verbindung der Formel (I) durch das Verfahren nach einem der Ansprüche 1 bis 8 und Vermischen der Verbindung mit einem pharmazeutisch verträglichen Träger.